# EUROPEAN PATENT SPECIFICATION

(11) **EP 3 482 583 B1**
(45) Date of publication and mention of the grant of the patent: **12.10.2022**
(21) Application number: 16742193.2
(22) Date of filing: 06.07.2016
(51) Int. Cl.: A61B 5/00, G16H 40/67, H04W 4/38, H04W 84/18, A61B 5/01, A61B 5/021, A61B 5/024, A61B 5/08, H04W 4/35, H04W 36/00

(54) **TRANSFER OF A MONITORING RESPONSIBILITY**
ÜBERTRAGUNG EINER ÜBERWACHUNGSVERANTWORTUNG
TRANSFERT D'UNE RESPONSABILITÉ DE SURVEILLANCE

(43) Date of publication of application: 15.05.2019
(73) Proprietor: Telefonaktiebolaget LM Ericsson (PUBL), 164 83 Stockholm (SE); Ericsson Telekommunikation GmbH, 60528 Frankfurt (DE)
(72) Inventor: DINKELAKER, Tom, 61194 Niddatal (DE); LIOKUMOVICH, Gregory, 61440 Oberursel (DE); SCHOLZ, Heike, 64297 Darmstadt (DE); NIEMOELLER, Joerg, 19149 Sollentuna (SE)
(74) Representative: Zacco Sweden AB
(86) International application number: PCT/EP2016/065911
(87) International publication number: WO 2018/006951

(56) References cited:
- WO-A2-2015/127056
- US-A1- 2007 055 472
- US-A1- 2013 191 681
- US-A1- 2014 304 383
- US-A1- 2015 182 174

## Description

### Technical Field

The present disclosure generally relates to a technique for monitoring an object. More specifically, and without limitation, a method and a device are provided for monitoring an object by means of a network that comprises a plurality of nodes for monitoring the object.

### Background

In an exemplary embodiment, a person is in need of day and night monitoring, e.g., an elder or a sick person living alone. In another use case, a person such as a security guard or a security officer is exposed to potential hazards. A conventional device for remote patient monitoring (RPM) or man-down monitoring gathers data for vital signs in conjunction with location data. Further use cases may require that goods are protected against theft or monitored for constant transport conditions.

Conventional techniques for monitoring an object (including a person or goods) use a single sensor or a single device with few integrated sensors. For example, a RPM device may comprise one sensor for gathering vital signs of a user. Alternatively, a monitoring device may use satellite positioning to collect location data in order to derive or track the movement of goods or the person wearing the device.

Such existing monitoring techniques fall short in terms of accuracy and reliability. A sensor may fail to detect a vital sign due to interference or an invalidated assumption, e.g. when the person is not changing position because the person is asleep. In such cases, false alarms may be triggered. The false alarms cause manual inspections such as check-up calls. However, manual inspections are costly and may disturb the user, e.g. when the check-up call wakes the sleeping user.

WO 2015/127056 A2 describes networked smart wearable devices and methods for dynamic power management of multiple wearable devices. US 2013/0191681 A1 describes signal processing-based fault detection, isolation and remediation. US 2007/0055472 A1 describes methods and systems for transferring data in a wireless network. US 2015/0182174 A1 describes a method and a system to reduce false clinical alerts triggered by a patient monitoring system, the false clinical alerts being associated with a clinical condition of a patient. US 2014/0304383 A1 describes a method for clustering devices in machine-to-machine networks to minimize collisions.

### Summary

Accordingly, there is a need for a technique that monitors an object more reliably or more accurately at least in certain situations.

As to one aspect, a method of monitoring an object by a plurality of monitoring nodes of network is provided. Therein, responsibility for the monitoring may be assigned to a certain monitoring node. Depending on certain conditions, responsibility may be passed from that node to another monitoring node or vice versa. Such transfer or takeover of responsibility may be based on a determination and comparison of rank values actually assigned to the different nodes.

Such comparison may be performed or triggered by that monitoring node, by any of the other monitoring nodes or by any other network node.

The method may comprise or triggering a step of providing, in the network, a rank value for monitoring the object by the certain monitoring node; a step of comparing the rank value for monitoring the object by the node with rank values provided by other monitoring nodes in the network for monitoring the object, or receiving a result of the comparison. It may further comprise a step of selectively sending a corresponding message depending on the comparison.

The rank value may be a function of the time and or of the location of the node and/or the monitored object. E.g. a rank value associated to a first node may be high at day time and low at night time, wherein a rank value of a second node may be low at day time and high at night time. It may further be dependent on the location such that the rank value decreases if the location is outside a certain location area.

Alternatively or additionally, the rank value may be dependent on a monitoring capability, such as a precision and/or reliability of measurements of an associated sensor.

Alternatively or additionally, the rank value may be dependent on a communication capability, e.g. a bandwidth, latency, and a reliability of a communication link involving the monitoring node.

Some or each of the steps of the method may be performed by a monitoring node. The method may be implemented at each of the nodes. The nodes may be in peer-to-peer communication via the network.

Alternatively or in addition, the method or at least some of the steps of the method may be performed by a server or central node in the network. For example, the monitoring nodes may receive the result of the comparison from the server. The step of comparing the rank values may be performed by the server for all monitoring nodes in the network.

The method may further comprise or trigger a step of receiving the rank values provided by the other nodes in the network for the comparison. Providing the rank value in the network may include sending the rank value to the server in the network for performing the comparison. The result of the comparison may be received from the server.

Based on the rank value, the responsibility for monitoring the object may be assigned and/or handed over to the node in the network, which monitors (or is capable of monitoring) the object with the highest accuracy and/or the highest reliability among all nodes in the network.

The method may further comprising or triggering a step of monitoring the object. The object may be monitored by the node, if the rank value for the node is greater than the rank values provided by the other nodes in the network.

Each of the nodes may comprise a sensor unit for monitoring the object. Monitoring the object may include (e.g., continuously or periodically) measuring values of the object. The message may include the measured values of the object and/or sending the message may further depend on the measured values.

The sensor unit may be configured to measure goods (as the object) or for medical monitoring. For example, the node may be implemented in a wristwatch, in clothes or in a mattress. The sensor unit may include any kind of sensors, e.g. acceleration sensors (for linear or angular acceleration). The node may be close to a person (as the object) to detect or derive body movements of the person. In another use case, goods are monitored, e.g., to ensure a continuous cooling chain for the goods.

The message may relate to the monitoring. The message may be triggered by monitoring the object. The message may include a result of monitoring the object. The monitoring may also be referred to as a measurement, a surveillance or an observation. The nodes for monitoring the object may also be referred to as sensor devices.

The step of providing the rank value may include calculating the rank (value), e.g., using several criteria.

At least some embodiments of the technique improve the quality of the monitoring by communicating with several sensor devices in the network, instead of using only one kind of sensor or using an isolated sensor device (e.g., at a single location). The nodes or sensor devices may communicate with each other, e.g. directly (by exchanging the rank values) or indirectly (when providing the rank values and receiving the comparison result, e.g., via the server that compares the rank values).

The step of comparing may include evaluating data (e.g., the rank values and/or monitoring values) of several nodes and/or combining the data of several nodes, e.g., to determine the node with the best surveillance quality. The node determined as a result of the comparison may take over the responsibility for the monitoring, e.g., in that the node is selected to send the message (e.g., if triggered by the monitoring).

For example, the step of sending the message may be subject to the condition that the node is selected (i.e., the node is responsible for monitoring the object) as the result of the comparison and that the monitoring of the object triggers the message. Sending the message may be subject to the condition that the rank value for monitoring the object by the node is greater than the rank values provided by each of the other nodes in the network for monitoring the object.

The node may keep the responsibility of monitoring and/or sending the message until another node in the network provides a rank value (e.g., indicating the best surveillance quality) that is greater than the current rank value of the currently responsible node. The rank value may be a function of a monitoring capability or quality. The monitoring capability or quality may be dependent on the time; e.g. the daytime. For instance, a first node (e.g., a wristwatch) comprising an imprecise gyro sensor in its sensor unit may be used during day time. During night time, a second node (e.g., implemented in a mattress) comprising a more precise motion sensor may be selected as the result of the comparison.

The node may implement a protocol for calculating the rank value and/or communicating with other nodes. In the context of its communication or networking capability, the node may also be referred to as an intelligent sensor device. The rank value may be provided and/or the message may be sent by broadcasting in the network (e.g., between the intelligent sensor devices). The step of providing the rank value in the network may include broadcasting the rank value to the other nodes in the network. By virtue of the rank values, the nodes may be configured for self-organization in the network, which is also referred to as a collaboration deployment.

The method may be performed by a wireless and/or wearable device. The node may be a wireless and/or wearable device. The node may be implemented in clothes or a wristwatch. The node may be a user equipment (UE) or a device for machine-type communication (MTC). The node may be powered by a battery unit. At least one of the step of providing the rank value, the step of sending the message and the step of receiving the result of the comparison may use optical (e.g., infrared) and/or radio communication.

At least one of the step of providing the rank value and the step of comparing the rank value may be performed in response to receiving a request for the comparison. The request for the comparison may also be referred to as a request for negotiation. The request for the comparison may be received from another node in the network and/or the server.

The method may further comprise or trigger a step of determining a state for the node. The state may depend on the comparison. The message may be selectively sent depending on the state.

The node may assume at least two different states. The node may assume binary states. The two states may be mutually exclusive.

The state may be indicative of whether or not the node is responsible for monitoring the object. The state may be indicative of whether or not the rank value for monitoring the object by the node is greater than the rank values provided by each of the other nodes in the network for monitoring the object. The node with the greatest rank value may assume a first state that indicates the responsibility for monitoring the object. All other nodes in the network may assume a second state that indicates the irresponsibility for monitoring the object.

The first state of the node may indicate or imply that the node is configured or allowed to send the message (e.g., if triggered by the monitoring). The second state of the node may indicate or imply that the node is configured to not send the message or is not allowed to send the message.

The object may be selectively monitored by the node depending on the state of the node. The node may cease monitoring the object in the second state, e.g., for energy efficiency. Alternatively, the node may (e.g., continuously or periodically) monitor the object in the second state (at least to an extent) for determining the rank value (e.g., the accuracy or reliability of measured values).

The method may further comprise or trigger a step of indicating (e.g., by means of broadcasting) the state to the other nodes in the network. The state may be indicated in response to the comparison. The state may be indicated to the other nodes in the network, if (e.g., only if) the rank value for the node is greater than the rank values of the other nodes.

The method may further comprise a step of broadcasting, in the network, the request for the comparison. The request may be broadcasted by the node, if the rank value of the node has changed. The request may be broadcasted, if the rank value of the node changes by more than a preset threshold value. The change of the rank value within a preset time period may be compared with the preset threshold value. The change may be a difference between the rank value at the beginning of the preset time period and the rank value at end of the preset time period. The threshold value for the change may specify a relative change of the rank value or an absolute change of the rank value. The relative change may be the change in relation to a previously provided rank value of the node or an average value of the rank values provided by all nodes in the network.

The request for the comparison may be broadcasted by the node, if the state of the node is indicative of the rank value for the node being greater than the rank values of the other nodes and the rank value of the node is declining. Alternatively or in addition, the request for the comparison may be broadcasted by the node, if the state of the node is indicative of the rank value for the node being not greater than the rank values of the other nodes and the rank value of the node is increasing.

The message may be a warning message. The step of sending the warning message may be further subject to the condition that the measured values are outside of a preset range. The object may include cargo. The measured values may include temperature values of the cargo.

Alternative or in addition, the object may include a person. The measured values may include a vital sign of the person. The measured values may include at least one of body temperature, blood pressure, pulse (or heart rate) and breathing rate (or respiratory rate) of the person. The object may be a patient. Alternatively or in addition, the warning message may be indicative of the absence of a vital sign for the person.

The method may further comprise or trigger a step of computing the rank value for monitoring the object by the node. The computation of the rank value of the node may include one or more of the following dependencies. The rank value may depend on an inverse distance or a proximity between the node and the object. The rank value of the node may depend on a quality of monitoring the object by means of the node, e.g., the accuracy and/or reliability of the monitoring. The rank value of the node may depend on at least one of a precision and a signal-to-noise ratio of values measured by the node. The signal may include, or may be indicative of, the measured values of the object. The noise may include, or may be indicative of, a variance of the measured values.

The rank value of the node may depend on a network connectivity of the node. The network connectivity may include a reliability and/or a signal strength of the node for communicating with the network. The rank value of the node may depend on availability or usage of resources at the node for the monitoring. The rank value of the node may depend on a cost function for using the resources for monitoring the object. The resources may include at least one of a battery level of the battery unit at the node and a processor of the node.

As to a further aspect, a computer program product is provided. The computer program product comprises program code portions for performing any one of the steps of the method aspects disclosed herein when the computer program product is executed by one or more computing devices. The computer program product may be stored on a computer-readable recording medium. The computer program product may also be provided for download via a data network, e.g., the network of nodes and/or the Internet.

As to another aspect, a device for selectively sending a message by a node in a network is provided. The network comprises a plurality of nodes for monitoring an object. The device comprises a providing unit configured to provide, in the network, a rank value for monitoring the object by the node; a comparing unit configured to compare the rank value for monitoring the object by the node with rank values provided by other nodes in the network for monitoring the object and/or configured to receive a result of the comparison; and a sending unit configured to selectively sending the message depending on the comparison.

Alternatively or in addition, the device may be configured to perform or trigger any of the steps of the method aspect.

As to a still further aspect, a node for selectively sending a message in a network is provided. The network comprises a plurality of nodes for monitoring an object. The node comprises a rank module for providing, in the network, a rank value for monitoring the object by the node; a comparison module for comparing the rank value for monitoring the object by the node with rank values provided by other nodes in the network for monitoring the object, and/or for receiving a result of the comparison; and a message module for selectively sending the message depending on the comparison.

The node may further comprise one or more modules for performing any of the steps of the method aspect.

As to a still further aspect, a network comprising a plurality nodes for monitoring an object is provided. Each of the nodes implements the method aspect and/or includes an embodiment of the device aspect.

The device, the node, the network and/or the server may further include any feature disclosed in the context of the method aspect.

### Brief Description of the Drawings

Further details of embodiments of the technique are described with reference to the enclosed drawings, wherein:
- Fig. 1: shows a schematic block diagram of a device for selectively sending a message by a node in a network;
- Fig. 2: shows a flowchart for a method of selectively sending a message by a node in a network, which is implementable by the device of Fig. 1;
- Fig. 3: shows a first embodiment of the device of Fig. 1 in a peer-to-peer network environment;
- Fig. 4: shows a second embodiment of the device of Fig. 1 in a centralized network environment;
- Fig. 5: shows exemplary rank values provided by the device of Fig. 1, 3 or 4 when performing the method of Fig. 2;
- Fig. 6: shows an exemplary signaling diagram resulting from performing the method of Fig. 2 in a peer-to-peer network environment;
- Fig. 7: shows an exemplary signaling diagram resulting from performing the method of Fig. 2 in a centralized network environment;
- Figs. 8A and 8B: schematically illustrate a comparative example and a network embodiment, respectively, in a first network scenario;
- Figs. 9A and 9B: schematically illustrate a comparative example and a network embodiment, respectively, in a second network scenario; and
- Fig. 10: shows an implementation of the device of any one of Figs. 1, 3 and 4.

### Detailed Description

In the following description, for purposes of explanation and not limitation, specific details are set forth, such as a specific network environment in order to provide a thorough understanding of the technique disclosed herein. It will be apparent to one skilled in the art that the technique may be practiced in other embodiments that depart from these specific details. Moreover, while the following embodiments are primarily described for short range networks such as IEEE 802.15 networks, the network communication may also be implemented using Bluetooth, Bluetooth Low Energy (BLE or Bluetooth smart), Z-Wave according to the Z-Wave Alliance, ZigBee based on the IEEE 802.15.4 radio standard, a Wireless Local Area Network (WLAN) according to the standard family IEEE 802.11 (e.g., IEEE 802.11a, g, n or ac), Long Term Evolution (LTE) according to 3GPP and/or a Worldwide Interoperability for Microwave Access (WiMAX) according to the standard family IEEE 802.16.

Moreover, those skilled in the art will appreciate that the modules, functions, steps and units explained herein may be implemented using software functioning in conjunction with a programmed microprocessor, an Application Specific Integrated Circuit (ASIC), a Field Programmable Gate Array (FPGA), a Digital Signal Processor (DSP) or a general purpose computer, e.g., including an Advanced RISC Machine (ARM). It will also be appreciated that, while the following embodiments are primarily described in context with methods and devices, the invention may also be embodied in a computer program product as well as in a system comprising a computer processor and memory coupled to the processor, wherein the memory is encoded with one or more programs that may perform the functions and steps and implement the modules and units disclosed herein.

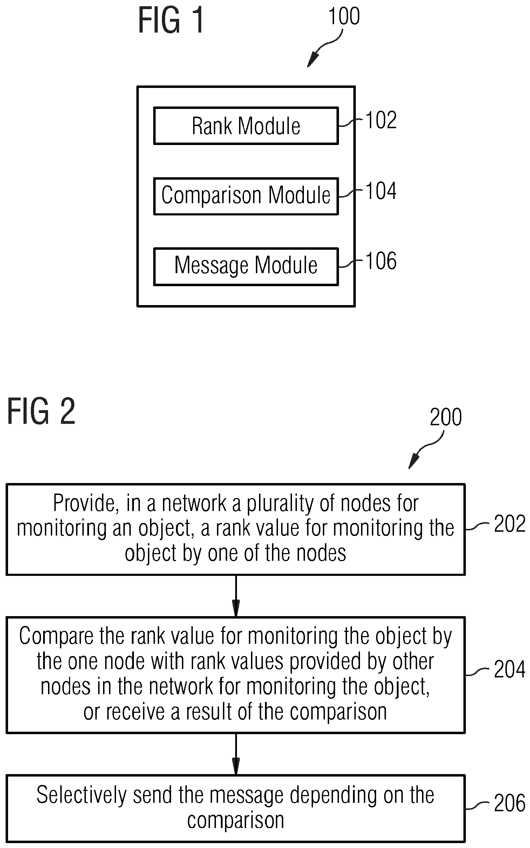

Fig. 1 schematically illustrates a device 100 for selectively sending a message by a node in a network. The device may be implemented at the node. The device and/or the node is configured to communicate through the network with other nodes of the network, each of which is configured to monitor an object.

The device includes a rank module 102 for providing (e.g., distributing), within the network, a rank value for monitoring the object by means of the node. The rank value is associated with the node. A comparison module 104 compares the rank value representative for monitoring the object by means of the node with rank values provided by the other nodes in the network. Each of the rank values of the other nodes is associated with the corresponding one of the other nodes. Alternatively to comparing the rank values at the node, a result of the comparison is received at the node.

Each of the rank values is representative of a quality for monitoring the object by means of the associated node. A message module 106 selectively sends the message depending on the comparison.

Fig. 2 shows a flowchart for a method 200 of selectively sending a message by a node in a network. In a step 202, a rank value is provided in a network that comprises a plurality of nodes for monitoring an object. The rank value provided by the node is indicative of a quality for monitoring the object by means of the node.

In a step 204, the rank value for monitoring the object by means of the node is compared with rank values provided by other nodes in the network and/or a result of such a comparison is received at the node.

Depending on the comparison, the node with the highest quality for monitoring the object is selected to send the message in a step 206. As a consequence, the responsibility of monitoring the object is handed over from one node to another node, so that the node with the currently best monitoring quality is in charge of monitoring the object and, when appropriate, sends the message according to the step 206. The message may be a warning message that is triggered based on the monitoring performed by the node.

The device 100 may perform the method 200. For example, the step 202, 204 and 206 may be performed by the modules 102, 104 and 106, respectively.

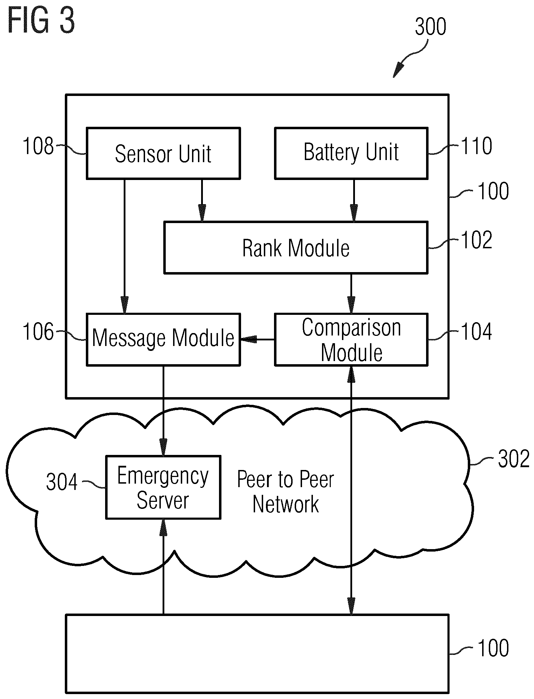

Fig. 3 shows a first embodiment of the device 100 in a peer-to-peer network environment 300. A network 302 couples a plurality of devices 100. Each of the devices 100 is implemented in one of the nodes of the network 302. The upper half of Fig. 3 shows a block diagram with exemplary components of one of the devices 100. The devices 100 may also be referred to as intelligent sensor devices as opposed to a conventional sensor device that is not capable of handing over the monitoring responsibility from one node to another node in the network 302.

Each of the devices 100 comprises a sensor unit 108 that is configured to monitor the object. At least some of the devices 100 are autonomously powered by energy harvesting or a battery unit 110. The rank value depends on the quality of monitoring the object by means of the sensor unit 108 and/or a reliability or a charging status of the power supply, e.g., the battery unit 110.

The rank value of the device 100 is computed by the rank module 102 and provided in the network 302 directly or (as illustrated in Fig. 3) through the comparison module 104.

The comparison module 104 maintains a state of the device 100. When the step 204 yields that the device 100 has the highest rank value compared to the rank values received from the other devices 100, the device 100 is in a first state. In the first state, the device 100 monitors the object and, where appropriate, sends the message according to the step 206. The message may be sent to another device 100 in the network 302 or to a server in the network 302. As illustrated in Fig. 3, the message is sent to an emergency server 304.

If the comparison step 204 yields that another device 100 has a higher rank value, the device 100 assumes a second state different from the first state. In the second state, no messages are sent according to the step 206.

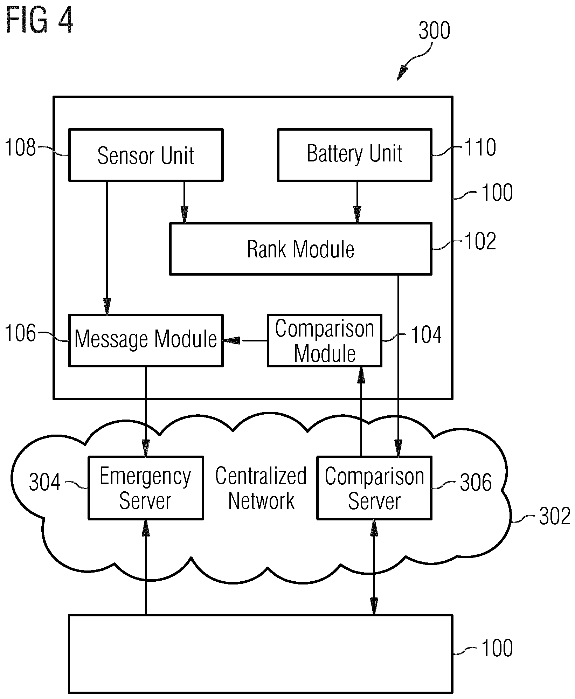

Fig. 4 shows a second embodiment of the device 100 in a centralized network 302. Features corresponding to those of the first embodiment are indicated by like reference signs.

The comparison of the rank values is performed in a centralized manner in the network 302, e.g., at a comparison server 306 that receives the rank values provided by the rank module 102 of each device 100. The comparison server 306 sends a result of the comparison, e.g., the state of the corresponding device 100, to each of the devices 100.

In any of above embodiments, several intelligent sensor devices 100 are used to improve the quality of the monitoring (e.g., a surveillance or an observation of a person or of commodities). The intelligent sensor devices 100 communicate with each other to rely upon the device 100 that currently achieves the best monitoring quality among the several devices 100 in the network 302.

The plurality of coupled devices 100 is also referred to as a monitoring system, e.g., a man-down system. Each of the devices 100 in the system may be arranged at different locations. The responsibility for the monitoring is handed over from one device 100 to another device 100 in the system. The responsibility is handed over according to the respective rank value. E.g., the device closest to the object is responsible for monitoring the object.

At least some of the devices 100 may be wirelessly connected to the network 302. For example, one of the devices 100 is implemented in a wristwatch of a person. Another device 100 may be implemented in a mattress for the person.

A conventional sensor node may comprise a sensor and a messaging component powered by a battery, and may be connected to a centralized network for independently sending warning messages. As illustrated in each of Figs. 3 and 4, in contrast to such a conventional sensor node, the intelligent sensor device 100 comprises additional components (e.g., the rank module 102 and the comparison module 104) and/or stores additional information (e.g., the state of the device 100).

The device 100 comprises, e.g., in the rank module 102, logic for calculating the rank value. The rank value defines a quality of monitoring the object (e.g., a measurement, a surveillance or an observation of the object). The rank value depends on several criteria. The input values for the calculating the rank values includes, e.g., measured values from the sensor unit 108 and/or a battery level of the battery unit 110. The device 100 determines the rank value by itself. E.g., each of the devices 100 evaluates the input values or the criteria independently.

The technique may be based on an exchange of rank values between the different devices 100 and logic, e.g., inside the rank module 102, to calculate and communicate the rank value that indicates the quality of the monitoring signal of the device 100 (e.g., of the sensor unit 108).

The device 100 performs a handover protocol, e.g. implemented in the comparison module 104, to send its data (e.g., its rank value and/or the values measured by its sensor unit 108) to the other devices 100 over a decentralized peer-to-peer network 302.

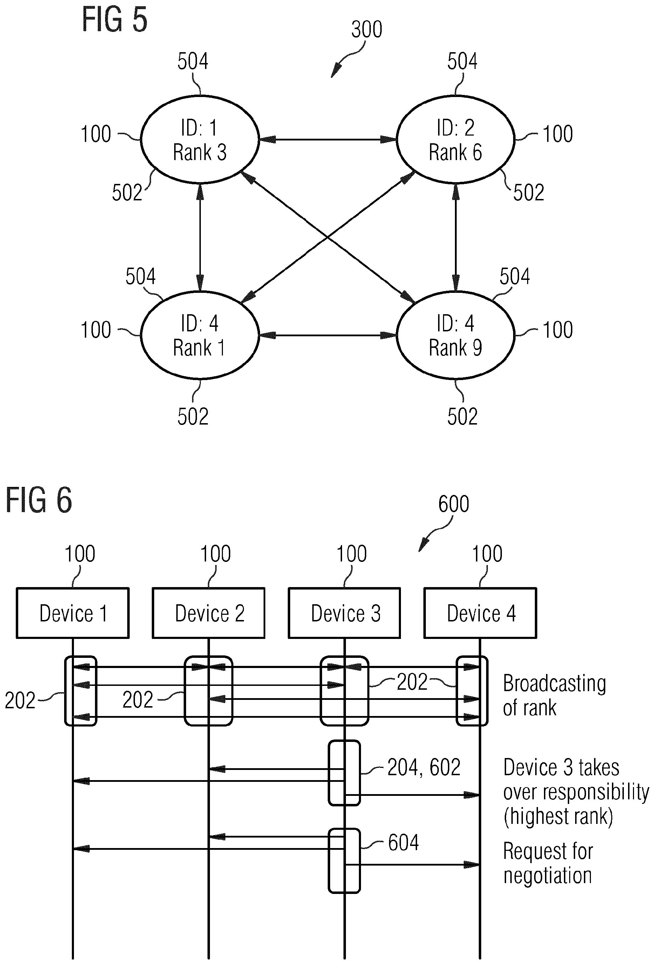

As schematically illustrated in Fig. 5, the messages that are exchanged according to the protocol include the rank value 502 of the device 100 (or the associated node) and an identifier 504 of the node or the device 100. In a collaboration deployment, every device 100 calculates its rank value 502 and communicates its rank value 502 with each other by broadcasting to all other peer devices 100.

Each device 100 periodically informs the others devices 100 about its rank value 502. The device 100 among the device 100 in the network 302 reporting the highest rank value takes over the responsibility (as the first state) and informs the others devices 100 about its state.

Fig. 6 shows a first example for a signaling sequence 600 in a network 302 including, by way of example, 4 implementations of the device 100. Steps of the method 200 are indicated by corresponding reference signs.

Furthermore, a step 602 of informing the other peer devices 100 as to taking over the responsibility (e.g., by assuming the first state) is performed by the one device 100 that determine in the step 204 of comparing the rank values that its rank value is the maximum among all devices 100 in the network 302.

In an implementation, the device 100 assumes the first state by receiving and/or storing a responsibility token at the device 100. The device 100 is responsible for triggering the alarm by sending the message according to the step 206 in the first state, e.g., if a positive surveillance signal of the sensor unit 108 is lost.

In specific situation, when the rank (value) of the responsible device 100 goes down and/or the rank of another (not responsible) device 100 significantly increases, a request for comparison (also referred to as a request for negotiation) is broadcasted in a step 604 by one of the devices 100 in the network 302. The request for comparison is a message that triggers a rank negotiation. For example, the step 604 of broadcasting the request triggers the steps 202 and 204 at each of the devices 100 in the network 302.

To ensure security and privacy, the devices 100 are preferably registered with each other by a shared secret (e.g., a personal identification number or PIN) and/or a shared public key. The registration may be verified at the beginning of each (e.g., bilateral) communication. Alternatively or in addition, the registration may be used for encrypting each communication. The public key may be certified and/or shared by means of a public-key infrastructure.

Alternatively or in combination, some or all messages exchanged between the devices 100, e.g., according to the signaling sequence 600, are implemented as one-way messages (which may also be referred to as fire-and-forget notifications), e.g., without awaiting an acknowledgment signal after each message.

The message for broadcasting the rank value according to the step 202 may be triggered by a timer, so that the step 202 is performed from time to time, e.g., periodically. Providing the rank value in the network 302 is implemented as a one-way message. Each device 100 broadcasts its current rank value to all other devices 100.

The broadcast message in the step 202 includes at least the rank value 502 and the identifier 504 of the device 100, e.g., a MAC address, a unique sequence number or a Globally Unique Identifier (GUID).

The rank value is a number resulting from the rank module 102. The highest rank value indicates the best monitoring quality.

Unique and accountable responsibility in the network 302 is controlled by means of the responsibility token that is only held by one of the devices 100 at any point in time. The responsibility token is passed on to another device 100, if the step 204 of comparing the rank values indicates that the other device 100 has the highest rank value. The device 100 that has, according to the rank value comparison 204, the highest rank value informs all other devices 100 in the step 602 that it has the responsibility token.

Any of the devices 100 is configured to trigger a new calculation of the rank value by broadcasting the request for negotiation in the step 604. The step 604 is triggered, if the rank value of the responsible device 100 goes dramatically down and/or the rank value of another device 100 improves, so that a new comparison 204 shall be performed.

The step 604 may be realized by means of a one-message communication. The one-message communication for the step 604 is followed by the message providing the rank value according to the step 202. Alternatively, a request-and-response message (i.e., a bi-directional communication) can be used. The response including the rank value 502 according to the step 202 is expected as the answer. The formerly responsible device 100 hands over the responsibility token to the device 100 with the highest rank value. By way of example, the signaling sequence 600 is repeated.

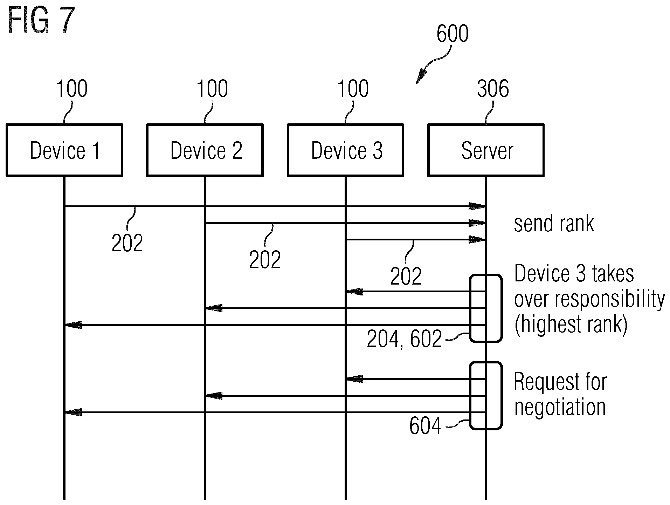

Fig. 7 shows a second example for a signaling sequence 600 resulting from a communication in the network 302 including, by way of example, 3 implementations of the device 100 and the server 306 for comparing the rank values. Steps corresponding to the method 200 and/or those of the first example for the signaling sequence are indicated by like reference signs.

The server 306 compares the rank values provided in the step 202. In the step 204, the server 306 signals the result of the comparison to the devices 100, as an implementation of the step 602.

The network 302 may be implemented by radio communication, e.g. according to Bluetooth or Wi-Fi. In one embodiment, the rank value is sent by a dedicated message format in the step 202. In another embodiment, the rank value is signaled in the step 202 by piggy-packing the rank value on existing protocols, i.e., by including the rank value in a header of the Wi-Fi protocol message (e.g., a beacon frame).

The continuous monitoring (e.g., the surveillance) is guaranteed as long as there is at least one device 100 monitoring the object at any point in time. The calculation of the rank value, e.g., in the step 202, may be based on any one of the following criteria. The criteria may be selected and/or defined in accordance with the monitoring task and/or the object to be monitored. The criteria may include at least one of connectivity of the device 100, precision of the sensor unit 108 for monitoring the object, a battery level of the battery unit 110, CPU usage cost at the device 100, a location of the device 100 and a proximity to the object.

In one embodiment, which is combinable with any embodiment of the device 100, the rank module 102 stores a weight for each of the criteria. The weight is applied in the calculation of the rank value of the corresponding device 100. The weight multiplied with its criterion defines a sub-rank. The rank calculation is based on these sub-ranks. Different formulas can be applied, e.g., depending on the device 100 and/or the monitoring task.

In a simple variant, the rank value is a (e.g., normalized) sum of all sub-ranks. More complicated (e.g., non-linear) formulas and/or time-dependent weights are applied for scenarios when a given sub-rank is critically important. For example, the critically important sub-rank can reset the resulting rank to 0 (e.g., even though all others sub-ranks are non-zero or high). This can be achieved by multiplication of all sub-ranks (as an example for a non-linear combination of the criteria).

Below table includes examples of the rank value calculation using a weight for computing each sub-rank and a multiplication for combining the sub-ranks.

| Criterion | Weight | Device 1 | Device 2 | Device 3 |
|---|---|---|---|---|
| Connectivity (e.g., in a range from 0 to 10) | 2 | 5 . 2 = 10 | 1 . 2 = 2 | 2 . 2 = 4 |
| Precision | 3 | 1 . 3 = 3 | 2 · 3 = 6 | 1 · 3 = 3 |
| Battery level | 3 | 5 . 3 = 15 | 1 · 3 = 3 | 10 . 3 = 30 |
| CPU usage cost | 2 | 1 · 2 = 2 | 1 · 2 = 2 | 2 · 2 = 4 |
| Proximity (e.g., in units of 1/m) | 1/x | 1/10 = 0.1 | 1/50 = 0.02 | 1/25 = 0.04 |
| Rank Value | | 90 | 1.44 | 57.6 |

The proximity criterion applies a nonlinear dependency, e.g., 1/x, for the distance x between the sensor unit 108 and the object.

In the example for the rank value in above table, the responsibility token is taken over by the Device 1.

The technique can be implemented to evaluate the rank values of individual devices 100 and to determine, based on the evaluation, the responsible device 100.

Each of the devices 100 may be type-specific. For example, a device 100 may include a gyro sensor in the sensor unit 108. The device 100 may be configured to detect movements. Same or another device 100 may include an air pressure sensor in the sensor unit 108. The device 100 may be configured to detect a change in altitude. Same or another device 100 may include a sensor unit 108 for measuring the pulse rate of a person. Devices 100 of different types may collaborate according to the technique. The type of the device 100 may be included as a criterion for calculating the rank value.

Further deployment scenarios and enhancements are described. The technique can achieve a self-organized collaboration deployment of the devices 100, e.g., using broadcast messages for the communication in the network 302. The technique can also be implemented or combined with other deployment scenarios.

A configuration setup is described. In case of including a further device 100 in the system, a request for negotiation is generated according to the step 604. The step 604 may be triggered manually or automatically by the presence of the further device 100, e.g., depending on requirements for configuration and security of the system. The request informs the other devices 100 about the identifier of the further device 100 and triggers broadcast messages including the rank values according to the step 202.

A device 100 that is excluded from the network 302 (which may also be referred to as signing off) shall not have the responsibility token. Before the device 100 signs off using a special message, the device 100 shall hand over the responsibility to another device 100.

In the centrally orchestrated deployment, the server 306 evaluates and communicates the responsibilities between the devices 100. The server 306 may take over a central integration point, e.g. from a conventional central server node.

The server 306 can be implemented in a smartphone, a dedicated device (e.g., arranged near the object, e.g., in a living room of the person) or a cloud service. The devices 100 send the rank information to the server 306 in the step 202 and receive the responsibility messages from the server 306 in the step 204. The server 306 decides which device 100 shall take over the responsibility and informs the devices 100 about this decision in the step 602.

The server 306 is the central point of the system. To avoid that the devices 100 cannot be managed if the server 306 fails, an advanced implementation uses a second server as a redundant server 306 replacing the failed server. Alternatively, the server 306 is avoided in the peer-to-peer network environment 300, e.g., as illustrated in Fig. 3.

In a variant, the server 306 for the orchestration of the devices 100 is integrated and/or implemented in one of the devices 100 or in several of the devices 100 for redundancy. For example, the functionality of the server 306 is performed by the device 100 with the highest rank, i.e., it takes over the responsibility for both monitoring the object and performing the centralized comparison. The responsibility remains with this device 100 as long as its server functionality is active and/or until the responsibility is delegated to another device 100 having a higher rank.

The responsible device 100 and/or the server 306 can trigger the request for negotiation according to the step 604. One advantage of such architecture is that the system can also work if the rank of the server 306 is low or no server 306 is available, because the functionality of the server 306 is seamlessly transferred to one of the devices 100 or the devices 100 switch to the peer-to-peer collaboration without the server 306.

In a cloud deployment of the technique, the devices 100 connect to a cloud service (e.g., provided in the network 302 or the Internet). The rank calculation in the step 202 is performed by the cloud service. Alternatively or in combination, the server 306 is run in the cloud.

The following figures schematically illustrate comparative examples comparing deployments with conventional sensors and deployments with intelligent sensors.

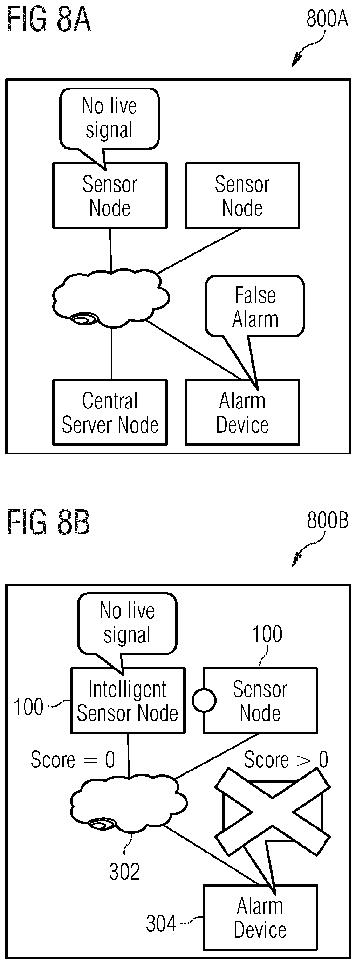

Figs. 8A and 8B schematically illustrate a first network scenario. In this network scenario, by way of example, one of the devices may fail to measure a vital signal. Consequently, the conventional system shown in Fig. 8A causes a false alarm. Without a rank value used in the steps 202 and 204 and the collaboration with handover according to the step 206, the conventional server-centric sensor network in Fig. 8A reports a false alarm. In contrast, the deployment of the devices 100 in Fig. 8B causes a handover to another peer device 100 (e.g., by taking over the responsibility token illustrated by the black dot) according to the method 200, since the rank value of the device 100 that fails to measure the vital signal is low, thus suppressing the potential false alarm.

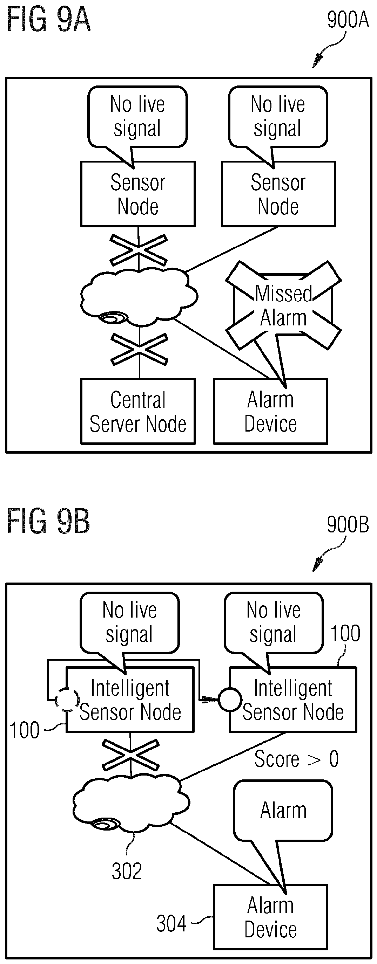

Figs. 9A and 9B schematically illustrate a second network scenario. When, by way of example, the connection to one conventional sensor or to the conventional central server node is lost, an alarm is missed, i.e. no alarm is triggered, as illustrated in Fig. 9A. In contrast, in the peer-to-peer network environment deploying the devices 100, the loss of a connection to one of the devices 100 triggers the handover protocol (as illustrated by the arrow in Fig. 9B), which ensures that an alarm is triggered as long as at least one device 100 is online.

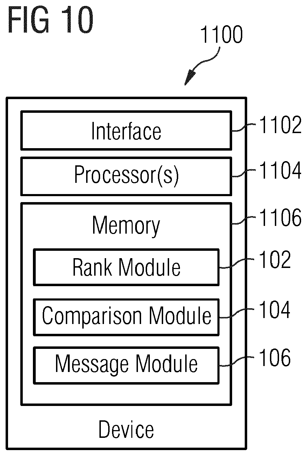

Fig. 10 shows an implementation 1100 of the device 100. The device 100 comprises one or more interfaces 1102 for communication with the sensor unit 106 and for communication via the network 302. The device 100 further comprises one or more processors 1104 operatively coupled to memory 1106. At least one of the rank module 102, the comparison module 104 and the message module 106 is implemented by performing the step 202, 204 and 206, respectively, using the one or more processors 1104 of the device 100. To this end, any of the modules 102, 104 and 106 may be encoded in the memory 1106.

As has become apparent from above description of exemplary embodiments, the monitoring quality, e.g., safety, reliability and/or accuracy of the monitoring, can be improved. At least in some implementations, sensor devices can prevent false and/or missed alarms. In same or other implementations, further sensor devices can be readily included in, and excluded from, a network environment.

In comparison to conventional server-centric sensor networks, the latency of the quality-based handover between the sensor devices is reduced. The sensor devices can be deployed without broadcasting for selecting a new coordinator in case the central server fails. The handover between the sensor devices can be triggered by a periodic request for comparison (e.g., after a preset time) and/or by an event-driven request for comparison (e.g., caused by a low battery status or a low communication bandwidth or signal quality, as reflected by the corresponding rank value).

Many advantages of the present invention will be fully understood from the foregoing description, and it will be apparent that various changes may be made in the form, construction and arrangement of the units and devices without departing from the scope of the invention and/or without sacrificing all of its advantages. Since the invention can be varied in many ways, it will be recognized that the invention should be limited only by the scope of the following claims.

## Claims

1. A method (200) in a network (302) comprising a plurality of nodes for monitoring an object, wherein a first node (100) of the plurality of nodes performs or triggers the steps of:
• providing (202), in the network (302), a first rank value (502) for monitoring the object by the first node (100);
• determining a comparison result by comparing (204) the first rank value (502) with at least a second rank value (502) provided by another node of the plurality of nodes, or receiving the comparison result from a server (306), wherein the first rank value (502) and the second rank value (502) are based on a monitoring capability and a communication capability of the first node (100) and the another node, wherein the monitoring capability comprises a precision and reliability of measurements by the first node (100) and the another node, and wherein the communication capability comprises: a bandwidth, latency, and a reliability of a communication link involving the first node (100) or the another node; and
• transferring or assuming a monitoring responsibility based on the comparison result.

2. The method (200) of claim 1, wherein the transfer of the monitoring responsibility is performed if the comparison result yields that the first rank value (502) is greater than the second rank value (502).

3. The method (200) of any of the preceding claims, wherein taking over the monitoring responsibility comprises sending a message to at least other nodes in the network (302).

4. The method (200) of any of the preceding claims, wherein taking over the monitoring responsibility comprises triggering an alarm if the monitoring yields certain conditions, e.g. if a value measured by the first node (100) meets a certain condition.

5. The method (200) of claims 2 to 4, wherein sending the message is subject to the condition that the first rank value (502) for monitoring the object by the first node (100) is greater than rank values provided by each of the other nodes of the plurality of nodes in the network (302) for monitoring the object.

6. The method (200) of any one of claims 1 to 5, further comprising or triggering the step of:
determining a state for the first node (100), wherein the state depends on the comparison result, and wherein the message is selectively sent depending on the state.

7. The method (200) of claim 5, wherein the state is indicative of whether or not the first rank value (502) for monitoring the object by the first node (100) is greater than the rank values (502) provided by each of the other nodes in the network (302) for monitoring the object.

8. The method (200) of claim 1, wherein a request (604) for comparison is broadcasted, if at least one of: the first rank value (502) of the first node (100) changes, e.g. if the first rank value (502) changes to a value equal or higher than that of rank values of all other nodes or if the rank values changes to a value equal or below the highest value of all the rank values of the other nodes.

9. The method (200) of any one of claims 1 to 7, wherein providing the first rank value (502) in the network (302) includes sending the first rank value (502) to the server (306) in the network (302) for performing the comparison, and wherein the result of the comparison is received from the server (306).

10. The method (200) of any one of claims 1 to 9, wherein the first rank value (502) of the first node (100) depends on at least one of:
• a time of monitoring the object,
• a location of the first node (100) or the object,
• a distance between the first node (100) and the object,
• a network connectivity of the first node (100),
• a use of resources at the first node (100),
• a cost function for using the resources for monitoring the object, and
• a signal-to-noise ratio associated to a communication link of the first node (100).

11. A computer program comprising program code portions for performing the steps of any one of claims 1 to 10 when the computer program is executed on one or more computing devices.

12. A computer program product, comprising the computer program of claim 11 stored on a computer-readable recording medium (1106).

13. A node (100) in a network (302) comprising a plurality of nodes for monitoring an object, the node (100) being configured to:
• provide (202), in the network (302), a first rank value (502) for monitoring the object by the node (100);
• determine a comparison result by comparing (204) the first rank value (502) with at least a second rank value (502) provided by another node of the plurality of nodes, or receive the comparison result from a server (306), wherein the first rank value (502) and the second rank value (502) are based on a monitoring capability and a communication capability of the node (100) and the another node, wherein the monitoring capability comprises a precision and reliability of measurements by the first node (100) and the another node, and wherein the communication capability comprises: a bandwidth, latency, and a reliability of a communication link involving the first node (100) or the another node; and
• transfer or assume a monitoring responsibility based on the comparison result.

14. The node (100) of claim 13, further configured to perform or trigger the steps of any one of claims 2 to 10.

## Patentansprüche

1. Verfahren (200) in einem Netzwerk (302), das eine Vielzahl von Knoten zum Überwachen eines Objekts umfasst, wobei ein erster Knoten (100) der Vielzahl von Knoten die folgenden Schritte ausführt oder auslöst:
• Bereitstellen (202) eines ersten Rangwerts (502) im Netzwerk (302) zum Überwachen des Objekts durch den ersten Knoten (100)
• Bestimmen eines Vergleichsergebnisses durch Vergleichen (204) des ersten Rangwerts (502) mit mindestens einem zweiten Rangwert (502), der von einem anderen Knoten der Vielzahl von Knoten bereitgestellt wird, oder Empfangen des Vergleichsergebnisses von einem Server (306), wobei der erste Rangwert (502) und der zweite Rangwert (502) auf einer Überwachungsfähigkeit und einer Kommunikationsfähigkeit des ersten Knotens (100) und des anderen Knotens basieren, wobei die Überwachungsfähigkeit eine Präzision und Zuverlässigkeit von Messungen durch den ersten Knoten (100) und den anderen Knoten umfasst, und wobei die Kommunikationsfähigkeit umfasst: eine Bandbreite, eine Latenz und eine Zuverlässigkeit einer Kommunikationsverbindung, die den ersten Knoten (100) oder den anderen Knoten einbezieht; und
• Übertragen oder Annehmen einer Überwachungsverantwortung auf Basis des Vergleichsergebnisses.

2. Verfahren (200) nach Anspruch 1, wobei das Übertragen der Überwachungsverantwortung durchgeführt wird, wenn das Vergleichsergebnis ergibt, dass der erste Rangwert (502) größer als der zweite Rangwert (502) ist.

3. Verfahren (200) nach einem der vorstehenden Ansprüche, wobei das Übernehmen der Überwachungsverantwortung das Senden einer Nachricht an mindestens andere Knoten in dem Netzwerk (302) umfasst.

4. Verfahren (200) nach einem der vorstehenden Ansprüche, wobei das Übernehmen der Überwachungsverantwortung das Auslösen eines Alarms umfasst, wenn das Überwachen bestimmte Bedingungen ergibt, z. B. wenn ein von dem ersten Knoten (100) gemessener Wert eine bestimmte Bedingung erfüllt.

5. Verfahren (200) nach Anspruch 2 bis 4, wobei das Senden der Nachricht der Bedingung unterliegt, dass der erste Rangwert (502) zum Überwachen des Objekts durch den ersten Knoten (100) größer ist als Rangwerte, die von jedem der anderen Knoten der Vielzahl von Knoten in dem Netzwerk (302) zur Überwachung des Objekts bereitgestellt werden.

6. Verfahren (200) nach einem der Ansprüche 1 bis 5, ferner den folgenden Schritt umfassend oder auslösend:
Bestimmen eines Zustands für den ersten Knoten (100), wobei der Zustand von dem Vergleichsergebnis abhängt und wobei die Nachricht selektiv abhängig vom Zustand gesendet wird.

7. Verfahren (200) nach Anspruch 5, wobei der Zustand angibt, ob der erste Rangwert (502) zum Überwachen des Objekts durch den ersten Knoten (100) größer als die Rangwerte (502) ist, die von jedem der anderen Knoten in dem Netzwerk (302) zum Überwachen des Objekts bereitgestellt werden.

8. Verfahren (200) nach Anspruch 1, wobei eine Anforderung (604) zum Vergleich gesendet wird, wenn sich mindestens eines von Folgendem ereignet: der erste Rangwert (502) des ersten Knotens (100) ändert sich, z. B. wenn sich der erste Rangwert (502) auf einen Wert ändert, der gleich oder höher ist als jene von Rangwerten aller anderen Knoten oder wenn sich die Rangwerte auf einen Wert ändern, der gleich oder unter dem höchsten Wert aller Rangwerte der anderen Knoten ist.

9. Verfahren (200) nach einem der Ansprüche 1 bis 7, wobei das Bereitstellen des ersten Rangwerts (502) in dem Netzwerk (302) das Senden des ersten Rangwerts (502) an den Server (306) in dem Netzwerk (302) zum Durchführen des Vergleichs umfasst und wobei das Ergebnis des Vergleichs von dem Server (306) empfangen wird.

10. Verfahren nach einem der Ansprüche 1 bis 9, wobei der erste Rangwert (502) des ersten Knotens (100) von mindestens einem der Folgenden abhängig ist:
• einer Zeit zum Überwachen des Objekts,
• einem Ort des ersten Knotens (100) oder des Objekts,
• einem Abstand zwischen dem ersten Knoten (100) und dem Objekt,
• einer Netzwerkkonnektivität des ersten Knotens (100),
• einer Verwendung von Ressourcen an dem ersten Knoten (100),
• einer Kostenfunktion zum Verwenden der Ressourcen zum Überwachen des Objekts und
• einem Signal-Rausch-Verhältnis, das einer Kommunikationsverbindung des ersten Knotens (100) zugeordnet ist.

11. Computerprogrammprodukt, umfassend Programmcodeanteile zum Durchführen der Schritte nach einem der Ansprüche 1 bis 10, wenn das Computerprogrammprodukt auf einer oder mehreren Computervorrichtungen ausgeführt wird.

12. Computerprogrammprodukt, umfassend das Computerprogramm nach Anspruch 11, das auf einem computerlesbaren Aufzeichnungsmedium (1106) gespeichert ist.

13. Knoten (100) in einem Netzwerk (302), umfassend eine Vielzahl von Knoten zum Überwachen eines Objekts, wobei der Knoten (100) konfiguriert ist zum:
• Bereitstellen (202) eines ersten Rangwerts (502) in dem Netzwerk (302) zum Überwachen des Objekts durch den Knoten (100);
• Bestimmen eines Vergleichsergebnisses durch Vergleichen (204) des ersten Rangwerts (502) mit mindestens einem zweiten Rangwert (502), der von einem anderen Knoten der Vielzahl von Knoten bereitgestellt wird, oder Empfangen des Vergleichsergebnisses von einem Server (306), wobei der erste Rangwert (502) und der zweite Rangwert (502) auf einer Überwachungsfähigkeit und einer Kommunikationsfähigkeit des ersten Knotens (100) und des anderen Knotens basieren, wobei die Überwachungsfähigkeit eine Präzision und Zuverlässigkeit von Messungen durch den ersten Knoten (100) und den anderen Knoten umfasst, und wobei die Kommunikationsfähigkeit Folgendes umfasst: eine Bandbreite, eine Latenz und eine Zuverlässigkeit einer Kommunikationsverbindung, die den ersten Knoten (100) oder den anderen Knoten einbezieht; und
• Übertragen oder Annehmen einer Überwachungsverantwortung auf Basis des Vergleichsergebnisses.

14. Knoten (100) nach Anspruch 13, der ferner dazu konfiguriert ist, die Schritte nach einem der Ansprüche 2 bis 10 auszulösen oder durchzuführen.

## Revendications

1. Procédé (200) dans un réseau (302) comprenant une pluralité de nœuds pour la surveillance d'un objet, dans lequel un premier noeud (100) de la pluralité de nœuds met en œuvre ou déclenche les étapes consistant à :
• fournir (202), dans le réseau (302), une première valeur de classement (502) pour la surveillance de l'objet par le premier noeud (100) ;
• déterminer un résultat de comparaison en comparant (204) la première valeur de classement (502) à au moins une deuxième valeur de classement (502) fournie par un autre noeud de la pluralité de nœuds, ou recevoir le résultat de comparaison depuis un serveur (306), dans lequel la première valeur de classement (502) et la deuxième valeur de classement (502) sont basées sur une capacité de surveillance et une capacité de communication du premier nœud (100) et de cet autre nœud, dans lequel la capacité de surveillance comprend une précision et une fiabilité de mesures par le premier noeud (100) et cet autre nœud, et dans lequel la capacité de communication comprend : une largeur de bande, une latence, et une fiabilité d'une liaison de communication impliquant le premier noeud (100) ou cet autre noeud ; et
• transférer ou assumer une responsabilité de surveillance sur la base du résultat de comparaison.

2. Procédé (200) selon la revendication 1, dans lequel le transfert de la responsabilité de surveillance est mis en œuvre si le résultat de comparaison aboutit à ce que la première valeur de classement (502) soit supérieure à la deuxième valeur de classement (502).

3. Procédé (200) selon l'une quelconque des revendications précédentes, dans lequel la prise en charge de la responsabilité de surveillance comprend l'envoi d'un message à au moins d'autres nœuds dans le réseau (302).

4. Procédé (200) selon l'une quelconque des revendications précédentes, dans lequel la prise en charge de la responsabilité de surveillance comprend le déclenchement d'une alarme si la surveillance aboutit à certaines conditions, par exemple si une valeur mesurée par le premier noeud (100) respecte une certaine condition.

5. Procédé (200) selon les revendications 2 à 4, dans lequel l'envoi du message est soumis à la condition que la première valeur de classement (502) pour la surveillance de l'objet par le premier nœud (100) soit supérieure à des valeurs de classement fournies par chacun des autres nœuds de la pluralité de nœuds dans le réseau (302) pour la surveillance de l'objet.

6. Procédé (200) selon l'une quelconque des revendications 1 à 5, comprenant ou déclenchant en outre l'étape consistant à :
déterminer un état pour le premier noeud (100), dans lequel l'état dépend du résultat de comparaison, et dans lequel le message est envoyé sélectivement en fonction de l'état.

7. Procédé (200) selon la revendication 5, dans lequel l'état est indicatif du fait de savoir si la première valeur de classement (502) pour la surveillance de l'objet par le premier noeud (100) est ou non supérieure aux valeurs de classement (502) fournies par chacun des autres nœuds dans le réseau (302) pour la surveillance de l'objet.

8. Procédé (200) selon la revendication 1, dans lequel une demande (604) de comparaison est diffusée, si au moins un parmi : la première valeur de classement (502) du premier noeud (100) change, par exemple si la première valeur de classement (502) change pour une valeur égale ou supérieure à celle de valeurs de classement de tous les autres nœuds ou si les valeurs de classement changent pour une valeur égale ou inférieure à la valeur la plus élevée de toutes les valeurs de classement des autres nœuds.

9. Procédé (200) selon l'une quelconque des revendications 1 à 7, dans lequel la fourniture de la première valeur de classement (502) dans le réseau (302) inclut l'envoi de la première valeur de classement (502) au serveur (306) dans le réseau (302) pour mettre en œuvre la comparaison, et dans lequel le résultat de la comparaison est reçu du serveur (306).

10. Procédé (200) selon l'une quelconque des revendications 1 à 9, dans lequel la première valeur de classement (502) du premier noeud (100) dépend d'au moins un parmi :
• un temps de surveillance de l'objet,
• une localisation du premier noeud (100) ou de l'objet,
• une distance entre le premier noeud (100) et l'objet,
• une connectivité réseau du premier noeud (100),
• une utilisation de ressources au niveau du premier noeud (100),
• une fonction de coût pour l'utilisation des ressources pour la surveillance de l'objet, et
• un rapport signal-bruit associé à une liaison de communication du premier noeud (100).

11. Programme informatique comprenant des parties de code de programme pour mettre en œuvre les étapes de l'une quelconque des revendications 1 à 10 lorsque le programme informatique est exécuté sur un ou plusieurs dispositifs informatiques.

12. Produit programme informatique, comprenant le programme informatique selon la revendication 11 stocké sur un support d'enregistrement lisible par ordinateur (1106).

13. Nœud (100) dans un réseau (302) comprenant une pluralité de nœuds pour la surveillance d'un objet, le noeud (100) étant configuré pour :
• fournir (202), dans le réseau (302), une première valeur de classement (502) pour la surveillance de l'objet par le noeud (100) ;
• déterminer un résultat de comparaison en comparant (204) la première valeur de classement (502) à au moins une deuxième valeur de classement (502) fournie par un autre noeud de la pluralité de nœuds, ou recevoir le résultat de comparaison depuis un serveur (306), dans lequel la première valeur de classement (502) et la deuxième valeur de classement (502) sont basées sur une capacité de surveillance et une capacité de communication du premier nœud (100) et de cet autre nœud, dans lequel la capacité de surveillance comprend une précision et une fiabilité de mesures par le premier noeud (100) et cet autre nœud, et dans lequel la capacité de communication comprend : une largeur de bande, une latence, et une fiabilité d'une liaison de communication impliquant le premier noeud (100) ou cet autre noeud ; et
• transférer ou assumer une responsabilité de surveillance sur la base du résultat de comparaison.

14. Nœud (100) selon la revendication 13, configuré en outre pour mettre en œuvre ou déclencher les étapes de l'une quelconque des revendications 2 à 10.
